# EUROPEAN PATENT APPLICATION

(11) **EP 4 218 539 A1**
(43) Date of publication of application: **02.08.2023**
(21) Application number: 22800566.6
(22) Date of filing: 07.01.2022
(51) Int. Cl.: A61B 1/317

(54) **SPINE INTERVERTEBRAL FUSION ENDOSCOPE STRUCTURE**

(30) Priority: 25.11.2021 CN 202111410941
(71) Applicant: Peking University Third Hospital, Beijing 100191 (CN); Shanghai Reach Medical Instrument Co.,Ltd, Shanghai 201114 (CN)
(72) Inventor: LI, Weishi, Shanghai 201114 (CN); HUANG, Xiaomin, Shanghai 201114 (CN); ZHONG, Woquan, Shanghai 201114 (CN); JIANG, Shuai, Shanghai 201114 (CN); BAI, Guanyu, Shanghai 201114 (CN); YE, Mingsha, Shanghai 201114 (CN)
(74) Representative: Sach, Greg Robert
(86) International application number: PCT/CN2022/070664
(87) International publication number: WO 2023/092831

(57) **Abstract**

Disclosed is a structure for endoscopic interbody spinal fusion, including: an implantation sleeve, an endoscope component closely fitting an inner wall of the implantation sleeve, and a mounting seat component fixedly connected to the endoscope component; the endoscope component includes an endoscope outer tube extending along a length of the implantation sleeve, as well as a water inlet pipe piece, a camera pipe piece in close proximity to the water inlet pipe piece, and a water outlet pipeline in close proximity to the camera pipe piece that are arranged side by side in the endoscope outer tube, where a water inlet pipe connector is connected to the water inlet pipe piece, and a water outlet pipe connector is connected to the water outlet pipeline; the water inlet pipe connector and the water outlet pipe connector are symmetrically arranged at two sides of the mounting seat component, and an eyepiece component is fixedly connected to the mounting seat component and is arranged corresponding to the camera pipe piece. The structure of the present invention is simple and scientific and can be used in combination with an endoscope in actual use; moreover, the structure expands the space for operation in a working channel, renders a clearer vision for operation in an aqueous medium, and improves the effect of the operation greatly.

## Description

### Technical Field

The present invention relates to the technical field of endoscopes, and more particularly, to a structure for endoscopic interbody spinal fusion.

### Background

Endoscopic spinal interbody fusion has been widely used in the treatment of lumbar disc herniation. Foraminal instruments are suitable for any operative approach, from either front or back, and left or right. Different approaches have distinct advantages, disadvantages, and indications, and the foraminal approach has the broadest application. Furthermore, the endoscope is suitable for fusion operation so that a cage can pass through a working channel with a minimum height. After the cage is placed in the proper position between the vertebral bodies, the height-adjustable cage is stretched and adjusted to a height suitable for the patient through the tools matched with the cage. Generally, the minimum height of the cage is set as the first height value.

In the case of the spinal interbody fusion endoscope in the prior art, an illumination light source, an optical fiber, and a cleaning water pipe are arranged inside one tube. Due to the limited space in the working channel, an operative tool and spinal a fusion endoscope must alternately appear in the working channel, that is, firstly, an operative tool is operated in the working channel in an invisible situation, and then the operative tool is taken out and the endoscope is inserted to check the operation situation. Repeated as such, the complexity of the operation is increased. Moreover, the existing endoscope in use is troubled by the limited space in the working channel, which is inconvenient for the operation; the endoscope is relatively long, which is inconvenient for an operator to apply force.

### Summary of the Invention

To address the defects in the prior art, it is an object of the present invention to provide a structure for endoscopic interbody spinal fusion, which is simple and scientific and can be used in combination with an endoscope in actual use; moreover, the structure expands the space for operation in a working channel, renders a clearer vision for operation in an aqueous medium, and improves the effect of the operation greatly. To achieve the above object and other advantages according to the present invention, a structure for endoscopic interbody spinal fusion s provided, including:
an implantation sleeve, an endoscope component closely fitting an inner wall of the implantation sleeve, and a mounting seat component fixedly connected to the endoscope component, wherein
the endoscope component includes an endoscope outer tube extending along a length of the implantation sleeve, as well as a water inlet pipe piece, a camera pipe piece in close proximity to the water inlet pipe piece, and a water outlet pipeline in close proximity to the camera pipe piece that are arranged side by side in the endoscope outer tube; a water inlet pipe connector is connected to the water inlet pipe piece, and a water outlet pipe connector is connected to the water outlet pipeline;
the water inlet pipe connector and the water outlet pipe connector are symmetrically arranged at two sides of the mounting seat component, and an eyepiece component is fixedly connected to the mounting seat component and is arranged corresponding to the camera pipe piece.

Preferably, the implantation sleeve includes a working sleeve, an endoscope transition sleeve fixedly connected to the working sleeve, and an endoscope outer tube guide sleeve provided at a lower end of the endoscope transition sleeve, wherein the endoscope outer tube passes through the endoscope outer tube guide sleeve.

Preferably, an end surface of the endoscope outer tube has an arc-shaped section, and the endoscope component closely fits the inner wall of the implantation sleeve so that the working sleeve and the endoscope outer tube form a working space.

Preferably, the mounting seat component includes an open mounting base and an extended flat seat integrally connected to the mounting base, wherein three through holes are provided in the middle of the extended flat seat, and the through holes are in one-to-one correspondence with the water inlet pipe piece, the camera pipe piece, and the water outlet pipeline piece, respectively.

Preferably, a water inlet port channel and a water outlet port channel are respectively opened on two opposite sides of the mounting base, and the water outlet pipe connector and the water inlet pipe connector are fixedly connected to the water inlet port channel and the water outlet port channel, respectively.

Preferably, a cover plate is fixedly connected to an opening of the mounting base, and an optical fiber interface is fixedly connected to an end face of the mounting base away from the cover plate.

Preferably, the endoscope outer tube guide sleeve is axially rotatable about the working sleeve.

Preferably, a total length of the endoscope outer tube plus the mounting seat component ranges from 100 cm to 300 cm.

Preferably, the endoscope outer tube is configured to isolate the water inlet pipe piece, the camera pipe piece, and the water outlet pipeline from the working channel for endoscopic visualization.

The present invention is more advantageous than the prior art because the water inlet pipe piece, the water outlet pipeline piece, and the camera pipe piece are installed in the implantation sleeve, whereby the volume of the endoscope system is reduced, and the endoscope system closely fits the inner wall of the implantation sleeve, which greatly expands the space of operation and is more suitable for various instruments; given the same diameter of the implantation sleeve, the space for operation obtained by using this endoscope is larger, and the adaptability to conventional instruments expands the movable space of an operative knife; more importantly, an expanded space does not cause a larger incision to the patient, that is, a minimal incision and increased freedom of operation are both ensured; moreover, the endoscope enables operation in aqueous medium, which is convenient for an operator to clearly observe the structure of spine; an unobstructed view in the operation is possible, which reduces the difficulty of operation, increases the decompression range of spinal operation, and improves the treatment effect.

### Brief Description of the Drawings

FIG. 1 is a schematic three-dimensional view of a structure for endoscopic interbody spinal fusion according to the present invention;
FIG. 2 is a three-dimensional exploded view of the structure for endoscopic interbody spinal fusion according to the present invention;
FIG. 3 is a front view of the structure for endoscopic interbody spinal fusion according to the present invention.

### Detailed Description of the Preferred Embodiments

The embodiments of the present invention will now be described more fully hereinafter with reference to the accompanying drawings, in which embodiments of the invention are shown. It is to be understood that the embodiments described are only some, but not all embodiments of the invention. Based on the embodiments of the present invention, all other embodiments obtained by a person of ordinary skill in the art without inventive effort fall within the scope of the present invention.

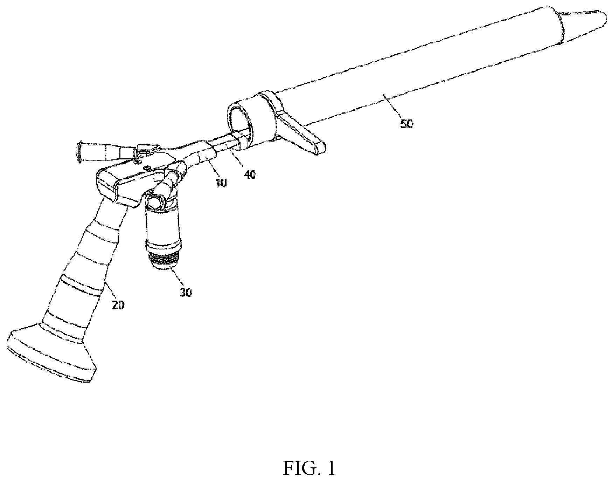

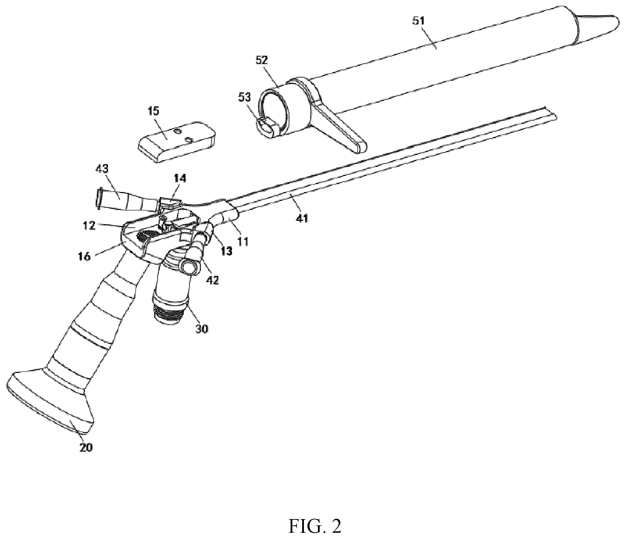

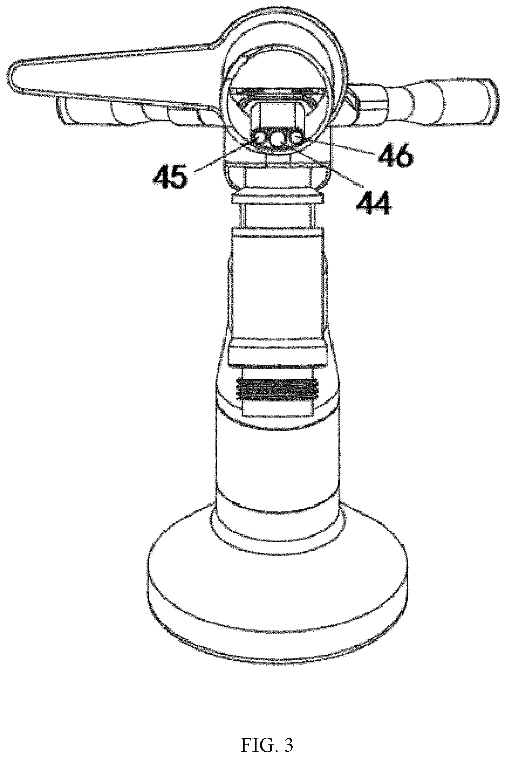

With reference to FIGS. 1 to 3, a structure for endoscopic interbody spinal fusion is shown, including: an implantation sleeve 50, an endoscope component 40 closely fitting the inner wall of the implantation sleeve 50, and a mounting seat component 10 fixedly connected to the endoscope component 40; the endoscope component 40 includes an endoscope outer tube 41 extending along a length of the implantation sleeve 50, as well as a water inlet pipe piece 45, a camera pipe piece 44 in close proximity to the water inlet pipe piece 45, and a water outlet pipeline 46 in close proximity to the camera pipe piece 44 that are arranged side by side in the endoscope outer tube 41, where a water inlet pipe connector 42 is connected to the water inlet pipe piece 45, and a water outlet pipe connector 43 is connected to the water outlet pipeline 46; the water inlet pipe connector 42 and the water outlet pipe connector 43 are symmetrically arranged on two sides of the mounting seat component 10, and an eyepiece component 20 is fixedly connected to the mounting seat component 10 and is arranged corresponding to the camera pipe piece 44. During use, the structure herein works in an aqueous medium by means of the water outlet pipeline 46 and the water inlet pipe piece 45 so that the operator can clearly see the spine and avoid a blurred view; moreover, the endoscope outer tube 41 closely fits the inner wall of the implantation sleeve 50, hence the space for operation in the implantation sleeve 50 is greatly expanded, which is more convenient for operation, applicable to various medical devices, and is more universal.

Furthermore, the implantation sleeve 50 includes a working sleeve 51, an endoscope transition sleeve 52 fixedly connected to the working sleeve 51, and an endoscope outer tube guide sleeve 53 provided at a lower end of the endoscope transition sleeve 52, where the endoscope outer tube 41 passes through the endoscope outer tube guide sleeve 53.

Furthermore, an end surface of the endoscope outer tube 41 has an arc-shaped section, and the endoscope component closely fits the inner wall of the implantation sleeve 50, whereby the working sleeve 51 and the endoscope outer tube 41 form a working space that is larger than that in the prior art.

Furthermore, the mounting seat component 10 includes an open mounting base 16 and an extended flat seat 11 integrally connected to the mounting base 16, wherein three through holes are provided in the middle of the extended flat seat 11, and the through holes are in one-to-one correspondence with the water inlet pipe piece 45, the camera pipe piece 44, and the water outlet pipeline 46, respectively; a water inlet port channel and a water outlet port channel are respectively provided on two opposite sides of the mounting base 16, and the water inlet port channel and water outlet port channel are fixedly connected to the water outlet pipe connector 43 connected to the water inlet pipe connector 42, respectively.

Furthermore, a cover plate 15 is fixedly connected to an opening of the mounting base 16, and an optical fiber interface 30 is fixedly connected to an end face of the mounting base 16 away from the cover plate 15; an optical fiber in the endoscope enters the camera pipe piece 44 and is connected to a camera head via the optical fiber interface 30 so that the operator has an unobstructed view in the whole process.

Furthermore, the endoscope outer tube guide sleeve 53 is axially rotatable about the working sleeve 51.

Furthermore, a total length of the endoscope outer tube 41 plus the mounting seat component 10 ranges from 100 cm to 300 cm; in an embodiment, the total length of the endoscope outer tube 41 plus the mounting seat component 10 is 190 cm, which is much smaller than a length of an endoscope in the prior art and facilitates the operation; moreover, a smaller length of the endoscope is more convenient for an operator to apply force.

Furthermore, the endoscope outer tube 41 is configured to isolate the water inlet pipe piece 45, the camera pipe piece 44, and the water outlet pipeline 46 from the working channel for visualizing endoscopic operation.

The number of devices and the scale of processing herein are intended to simplify the description of the invention, and applications, modifications, and variations of the invention will be apparent to those skilled in the art.

Although embodiments of the present invention have been disclosed above, the present invention is not limited to the applications set forth in the specification and embodiments. The present invention can be fully applied to various fields suitable for the present invention. Additional modifications can readily be implemented by those skilled in the art. Therefore, the invention can include others than the specific details and illustrations herein shown and described, without departing from the general concept defined by the appended claims and the scope of equivalents.

## Claims

1. A structure for endoscopic interbody spinal fusion, **characterized by** comprising:
an implantation sleeve (50), an endoscope component (40) closely fitting an inner wall of the implantation sleeve (50), and a mounting seat component (10) fixedly connected to the endoscope component (40), wherein
the endoscope component (40) comprises an endoscope outer tube (41) extending along a length of the implantation sleeve (50), as well as a water inlet pipe piece (45), a camera pipe piece (44) in close proximity to the water inlet pipe piece (45), and a water outlet pipeline (46) in close proximity to the camera pipe piece (44) that are arranged side by side in the endoscope outer tube (41); a water inlet pipe connector (42) is connected to the water inlet pipe piece (45), and a water outlet pipe connector (43) is connected to the water outlet pipeline (46);
the water inlet pipe connector (42) and the water outlet pipe connector (43) are symmetrically arranged at two sides of the mounting seat component (10), and an eyepiece component (20) is fixedly connected to the mounting seat component (10) and is arranged corresponding to the camera pipe piece (44).

2. The structure for endoscopic interbody spinal fusion according to claim 1, **characterized in that** the implantation sleeve (50) comprises a working sleeve (51), an endoscope transition sleeve (52) fixedly connected to the working sleeve (51), and an endoscope outer tube guide sleeve (53) provided at a lower end of the endoscope transition sleeve (52), wherein the endoscope outer tube (41) passes through the endoscope outer tube guide sleeve (53).

3. The structure for endoscopic interbody spinal fusion according to claim 2, **characterized in that** an end surface of the endoscope outer tube (41) has an arc-shaped section, and the endoscope component closely fits the inner wall of the implantation sleeve (50) so that the working sleeve (51) and the endoscope outer tube (41) form a working space.

4. The structure for endoscopic interbody spinal fusion according to claim 1, **characterized in that** the mounting seat component (10) comprises an open mounting base (16) and an extended flat seat (11) integrally connected to the mounting base (16), wherein three through holes are provided in the middle of the extended flat seat (11), and the through holes are in one-to-one correspondence with the water inlet pipe piece (45), the camera pipe piece (44) and the water outlet pipeline piece (46), respectively.

5. The structure for endoscopic interbody spinal fusion according to claim 4, **characterized in that** a water inlet port channel and a water outlet port channel are respectively opened on two opposite sides of the mounting base (16), and the water outlet pipe connector (43) and the water inlet pipe connector (42) are fixedly connected to the water inlet port channel and the water outlet port channel, respectively.

6. The structure for endoscopic interbody spinal fusion according to claim 4, **characterized in that** a cover plate (15) is fixedly connected to an opening of the mounting base (16), and an optical fiber interface (30) is fixedly connected to an end face of the mounting base (16) away from the cover plate (15).

7. The structure for endoscopic interbody spinal fusion according to claim 2, **characterized in that** the endoscope outer tube guide sleeve (53) is axially rotatable about the working sleeve (51).

8. The structure for endoscopic interbody spinal fusion according to claim 3, **characterized in that** a total length of the endoscope outer tube (41) plus the mounting seat component (10) ranges from 100 cm to 300 cm.

9. The structure for endoscopic interbody spinal fusion according to claim 1, **characterized in that** the endoscope outer tube (41) is configured to isolate the water inlet pipe piece (45), the camera pipe piece (44), and the water outlet pipeline (46) from the working channel for visualizing endoscopic operation.
